# EUROPEAN PATENT APPLICATION

(11) **EP 3 181 103 A1**
(43) Date of publication of application: **21.06.2017**
(21) Application number: 15200437.0
(22) Date of filing: 16.12.2015
(51) Int. Cl.: A61F 13/00, A61F 13/02, A61F 13/08

(54) **MEDICAL ARTICLE AND SKIN-ADHERING BANDAGE**

(71) Applicant: 3M Innovative Properties Company, St. Paul, MN 55133-3427 (US)
(72) Inventor: Hitschmann, Mr. Guido, 41470 Neuss (DE); Dietrich, Stefan Oliver, 40878 Ratingen (DE); Rule, Joseph Douglas, Woodbury, MN 55125 (US); Rogers, John Joseph, Saint Paul, MN 55119 (US); Bainbridge, Paul, Nottingham, NG10 3FX (GB)
(74) Representative: Müller, Bruno

(57) **Abstract**

The present invention pertains to a medical article (1) for use in compression therapy, comprising a continuous compression film (2) for application around the limb to provide compression of the limb and an adhesive (3) covering at least a portion of a first surface (4) of the continuous compression film, the adhesive being configured such as to skin-attach the continuous compression film. The continuous compression film has viscoelastic properties in at least one stretch direction (6, 6') in the plane of the continuous compression film. The present invention particularly pertains to a skin-adhering bandage for compression therapy comprising such a medical article.

## Description

The present invention pertains to a medical article for use in compression therapy, in particular compression therapy of a patient's limb, and a skin-adhering bandage comprising such a medical article.

Compression bandages are a common compression system used for medical compression therapy. They are utilized in the treatment of edema, as well as venous and lymphatic disorders, e.g. of the lower limbs. For example, compression bandages are considered useful in the management and treatment of chronic wounds, such as venous leg ulcers. In a further area, a compression bandage may be employed on a patient's leg after a foam treatment of superficial varicosities. As a result, vein regression may be significantly improved in many cases. In addition, such a bandage may be conceived to be more comfortable than, for example, compression stockings. As this kind of bandage is often worn over a period of several weeks, the bandage may be configured to be water resistant (e.g. for showering) and transparent (e.g. for visual wound inspection).

A prerequisite for applying a compression bandage in the treatment of lymphedema or venous leg ulcer is that the bandage provides sufficiently high pressure on the limb, especially in case of standing or movement. Once applied, stretch resistance of the compression bandage may ensure that a good, i.e. relatively large, static stiffness index is obtained. Static stiffness index is the difference in pressure below the compression bandage between a standing position and a supine position. A high static stiffness index results in the venous muscle pump working well so that edema may be reduced and ulcers may heal. Further, in case of edema reduction, there is a need to adapt to decreasing volumes of the limb to be able to provide sufficient pressure throughout the therapy.

In the context of the present disclosure, the term "stiffness" refers to the stretch resistance of a compression device when the compression device is elongated or stretched as it is when applied on a patient's limb.

The use of conventional compression bandages generally often involves the application of a multilayer compression bandage with elastic properties. One concept behind a number of such multi-layer bandaging systems is the use of a combination of different types of bandage layers in order to apply pressure in layers (providing an accumulation of pressure) and to achieve sustained compression together with rigidity. To ensure the desired pressure profile in the application of elastic compression bandages, as for example disclosed in document WO 2015/124669 A1, it may be required to provide a high and/or fine-tuned (pre-)stretch and/or tension of the bandage, which may be difficult to accomplish for the therapist. To assure proper and effective compression bandaging, it may be necessary that a medical professional applies the bandages, which may be regarded as inconvenient, in particular if bandages have to applied repeatedly, e.g. due to changes in pressure (e.g. reduction of pressure) and/or in uniformity of pressure.

It is one object of the present invention to provide cost-efficient solutions for compression therapy that feature high efficacy and enable convenient and simple application.

This object is achieved by a medical article having the features of claim 1 and/or a skin-adhering bandage having the features of claim 15.

According to a first aspect of the invention, a medical article for use in compression therapy of a patient's limb comprises a continuous compression film for application around the limb to provide compression of the limb. The medical article further comprises an adhesive covering at least a portion of a first surface of the continuous compression film. The adhesive is configured such as to skin-attach the continuous compression film. The continuous compression film has viscoelastic properties in at least one stretch direction in the plane of the continuous compression film.

According to a second aspect of the invention, a skin-adhering bandage for compression therapy comprises a medical article according to the first aspect of the invention.

An idea of the present invention is to utilize a single continuous viscoelastic film for compression instead of a conventional multi-layer bandaging system with one or several purely elastic layers and additional support layers. Such a viscoelastic film provides various advantages. It may provide a strain-rate dependent tensile modulus (i.e. stretch resistance in the stretch direction) and thus may combine high comfort in long-term, low pressure conditions with high, short-term stretch resistance or stiffness. Viscoelastic materials are able to adapt to body contours very well and thus may provide an excellent close fit with low pressure, e.g. in a supine position. Furthermore, the film is able to adapt to shrinking limb contours, e.g. in case of edema reduction. Since the viscoelastic materials provides resistance against rapid movements and/or high strain rates, a high static stiffness index may be achieved, which consequently implies a good venous muscle pump efficacy. In other words, the sub-bandage pressure amplitudes will be high during walking, which will support the muscle pump.

In addition to these advantages, the application of a medical article according to the invention may be simpler than that of a conventional, purely elastic bandage, especially when applied in a pre-stretched mode on a roll. A fine tuning of a (pre-)tension is not required for the continuous compression film and the article may be applied in a broad range of stretch ratios. Further, a single continuous viscoelastic film may be manufactured in very thin configurations and thus is very cost- and weight-efficient. Additional support layers or similar are not required. Thus, the solution according to the invention also may save waste in a significant way, as liners or support layers conventionally employed for the application of many traditional compression bandages are not needed. As a result, the medical article according to the invention is inexpensive, easy to use, and combines efficacy with a high level of comfort. A transparent medical article according to one aspect of the invention may not require changing so frequently, because no removal is necessary to inspect the skin underneath.

The medical article of the present invention may be included in or be employed in compression bandages, compression wraps, compression dressings, compression devices or the like. Such compression bandages may be used in compression therapy for applying compression to a body part, in particular a limb, of a subject for the use in the treatment and/or management of chronic wounds, edema and other venous and lymphatic disorders, more particularly venous leg ulcers and lymphedema of a limb. However, the medical article according to the invention may also be employed to treat other body parts than limbs, e.g. a torso, head, neck and so on. The continuous compression film may have viscoelastic properties mainly in one single specific stretch direction or in several, in particular independent, stretch directions.

Advantageous aspects and improvements of the present invention emerge from the further dependent claims and from the description with reference to the figures of the drawings.

According to an aspect of the invention, the medical article may have, in the stretch direction, a tensile modulus, which increases with increasing strain rate. Hence, the continuous compression film may allow the resulting medical article to exhibit a more rigid, stiff, or less compliant behavior in response to higher, i.e. faster, strain rates and a softer, more compliant behavior in response to lower, i.e. slower, strain rates. Examples of higher strain rates may occur, for example, when a subject is walking or running. Lower strain rates may occur, for example, as a result of allowing the subject to stand (e.g. if the medical article is wrapped around the lower leg), to sit (e.g. if the medical article is wrapped around a knee that is bent in the sitting position), to have de-swelling (or decreasing edema), or otherwise produce a lower strain rate on the medical article. The medical article and/or bandage generally may act as a relatively stiff (e.g. non-stretchable) sleeve to enable the muscle pump mechanism. The bandage generally may be tight enough to stay in place on a body part, but not so tight that it is uncomfortable or even unsafe to wear for extended periods.

Conventional (purely elastic) compression bandages may principally be able to achieve some of these properties through a non-linear force-elongation behavior upon stretching, as long as the bandage is applied at "full stretch", i.e. the degree of elongation that is the transition between an elastic, low modulus section and a high modulus section. The high modulus section may result, for example, from the stress-strain properties of a non-stretchable component employed in the bandage. In cases where such bandages are applied with too little stretch (or in cases of edema volume loss, which can occur, for example, within in the first few hours of the application), the compression provided by the bandage might be too low to adequately meet the compression needs. In contrast, medical articles of the present disclosure may provide similar proper compression therapy behavior, but at a relatively broad range of stretch ratios, where the ratio of stretched length to relaxed length is referred to as stretch ratio. As a result, medical articles, and particularly bandages, of the present disclosure can enable extended compression therapy and support the muscle pump even where edema are rapidly decreasing in size. In addition, medical articles of the present disclosure can enable less-skilled caregivers to safely and effectively apply the medical articles because, compared to existing medical articles, the short-term resistance to stretch is relatively independent of the stretch ratio upon application of the medical article to the limb.

A medical article of the present disclosure has a unique performance due to the continuous compression film employed in the medical article. The viscous nature of the continuous compression film particularly refers to its increasing resistance to elongation when it is stretched at high strain rates, i.e. at high speed. The overall effect is that the force required to stretch the medical article depends on both how fast the continuous compression film is being stretched and how far it has been stretched. This is different from (purely) elastic materials used in conventional compression bandages in which the force required to stretch the elastic material varies with how far the elastic material is stretched, but does not substantially vary with how fast the material is stretched.

The present inventors recognized that, in a compression therapy application, muscle contractions (amplitudes) that enable the muscle pump mechanism happen relatively rapidly, causing relatively high strain rates in a medical article employed. The present inventors further recognized that, in contrast, conformability to a limb, potential sloughing of the medical article (e.g. bandage), and de-swelling all happen relatively slowly, causing relatively low strain rates in the medical article employed. As a result, the present inventors discovered that medical articles that employ a continuous compression film of the present disclosure could exhibit a desirable, stiffer behavior in response to higher strain rates, while also exhibiting a desirable, more compliant behavior in response to lower strain rates.

The continuous compression film may have, for a sinusoidal deformation of the continuous compression film into the stretch direction in a dynamic mechanical analysis, a phase lag of δ between strain and stress, a storage modulus E' and a loss modulus E". Hereby tan δ = E"/E' > 0.3 may hold at maximum as a function of temperature. A dynamic mechanical analysis (DMA) is a method for studying the viscoelastic behavior of materials. DMA measures the viscoelastic moduli, that is the storage modulus E' and the loss modulus E", which together form a complex modulus, and tan δ of materials as they are deformed under a period (sinusoidal) deformation (stress or strain). For example, a sinusoidal stress may be applied and the strain in the material may be measured. For a perfectly elastic solid, the resulting strain and the stress will be perfectly in phase. For a purely viscous fluid, there will be a 90 degree phase lag of strain with respect to stress. Viscoelastic materials have characteristics between these two extremes, so that some phase lag will occur during DMA tests. The storage modulus E' measures the stored energy, representing the elastic portion, and the loss modulus E" measures the energy dissipated as heat, representing the viscous portion. The requirement tan δ = E"/E' > 0.3 thus means that the continuous compression film is required to have some minimal viscous properties above a certain lower limit, e.g. at room temperature of about 21°C. The temperature of the sample or the frequency of the stress may be varied, leading to variations in the complex modulus. With this approach the glass transition temperature of the material may be located, which defines a midpoint between the glassy (i.e. hard and relatively brittle state) and the rubbery (i.e. molten) states of a material. tan δ is generally a function of temperature and features a peak in a specific temperature range, where the maximum of the peak is around the transition region between glassy and rubbery states of the material.

The maximum of tan δ may define the glass transition temperature of the continuous compression film. The glass transition temperature may have values between 15 °C and 55 °C. The medical article and/or the skin-adhering bandage may thus particularly be applied under room temperature. The maximum of tan δ as a function of temperature is one of several possible parameters that may be used to define the glass transition temperature. The height and shape of the tan δ peak change systematically with amorphous content of the material.

At the glass transition temperature, the storage modulus E' may have values between 5 MPa and 500 MPa. Hence, on one hand, the continuous compression film must have a minimum storage modulus of at least E' > 5 MPa. And on the other hand, the continuous compression film should not provide too high compression forces, once the viscous component is no longer effective, i.e. E' < 500 MPa. The storage modulus E' may particularly have values between 5 MPa and 200 MPa.

According to an aspect of the invention, the medical article may be wound up in a roll in an application direction of the medical article. The medical article may thus be provided in a conveniently rolled configuration to ease the application as well as the packaging and the distribution of the medical article. The article may simply be unrolled directly onto the body part of a subject in the application direction, so that it can properly be applied by a less-skilled caregiver without having to rely on a medical professional. In this aspect, a machine direction (MD) of the medical article may be defined, which may generally correspond to the application direction. Consequently, also a cross-machine direction (CMD) may be defined, which may generally correspond to a direction perpendicular to the machine direction. Accordingly, a length of the medical article may defined as the dimension of the medical article in the MD, a width of the medical article may be defined as the dimension of the medical article in the CMD, and/or a thickness of the medical article may be defined as the dimension of the medical article in a direction oriented orthogonally with respect to the MD and the CMD. The medical article may further be protected by a husk or similar, e.g. a layer of paper fixed with some form of adhesive, to secure the roll from partial self-unwrapping or the like.

The medical article may be wound up under a pre-elongation in the application direction. The medical article may thus be elongated to a desired stretch ratio and rolled up while stretched such that it maintains its stretch ratio. The application direction may particularly be substantially equal to the stretch direction. The article can be unrolled directly onto the body part of a subject with negligible application of tension as the bandage is unrolled. With time, e.g. within seconds or minutes, due to its vis-coelastic properties, the medical article will recover and apply the amount of pressure to the subject that is determined by the stretch ratio applied to the article before it was rolled. This procedure would be more difficult with a conventional, strain-rate-independent elastic bandage because the bandage would immediately recover upon unrolling, so it would either lose its stretch or would have to be applied with noticeable tension. Because the medical articles of the present disclosure can be applied with negligible tension and still maintain the correct stretch ratio, it can properly be applied by a less-skilled caregiver. The attendance of a medical professional may no longer be necessary.

The pre-elongation in the application direction may amount to 130% to 250%, more preferably 150% to 190% of original length of the medical article in the application direction. In other words, the length of the medical article after pre-elongation is 130% to 250%, more preferably 150% to 190% of its original length, i.e. before pre-elongation. Pre-stretching and storing the medical article with the continuous compression film under such an elongation is expected to be a reasonable mode for convenient application.

According to an aspect of the invention, the medical article may further comprise a liner tab attached to at least one application end of the medical article in the application direction. In order to facilitate grabbing, e.g. when the medical article is unrolled onto the body part of a subject, and in order to keep the edges of the medical article uniformly stretched, such a liner tab may be provided at a roll end. The use of a liner may further ease the application of the medical article, e.g. the bandage. The liner tab may for example comprise a more or less rigid plastic material. Alternatively, the liner tab may also be configured as an elastic and/or viscoelastic film or layer, e.g. a foil or the like, on one or several ends of the medical article, e.g. the bandage. In one aspect, the liner tab covers at least between 0.5 cm and 10 cm of the adhesive in the application direction over substantially a width of the medical article perpendicular to the application direction.

An application of such a medical article may include unwinding approximately 20 centimeters of the roll, attaching the end of the medical article to a limb, e.g. starting approximately two centimeters away from the liner tab, pressing (adhering) approximately five centimeter of material, removing the liner tab under a 90° angle and/or adhering the end of the medical article to the limb. The method may comprise further unwinding and attaching the medical article to the limb. To this end, the medical article may be stretched slightly further if needed. For example, the medical article may be placed in a spiral pattern with an intermediate overlap of at least one millimeter. Finally, the medical article may be cut with scissors if the length is too long.

According to an aspect of the invention, the medical article may have a thickness of less than 40 µm in a non-stretched state. The medical article may particularly have a thickness of 30 µm in a non-stretched state. A non-stretched state may be defined for example as the state of the medical article after unrolling and waiting for specific period of time, e.g. several minutes, hours or days at room temperature. The person of skill will be aware that depending on the application, the used materials and the pre-elongation of the medical article different periods of time may be needed in order to reach a substantially non-stretched state. Due to the fact that the medical article comprises a single continuous compression film, which is at least partially covered by an adhesive, the medical article may be configured in a very thin way. The thickness of the film may be balanced out between several factors comprising the short-term (transient) and long-term (static) modulus for example. A further factor may be the water vapor permeability, i.e. the properties of the medical article as a moisture barrier. Further factors comprise the medical article's stability, its manageability during application, its costs and so on. These factors may also affect the choice of material of the continuous compression film, in particular the specific composition or formulation of the compression film. From a therapist point of view, it is advantageous to have the medical article wound up in a roll in pre-stretched mode. A further requirement may be that the continuous compression film does not easily tear and can be well grabbed and handled (e.g. using a liner tab attached to the medical article). These requirements determine a minimum thickness that the continuous compression film may have. The medical article may further have a width perpendicular to the application direction between 5 cm and 20 cm and may have a length in application direction of several decimeters up to several meters.

According to an aspect of the invention, at least one of the continuous compression film and the adhesive may comprise a visually transparent portion. A (partially) transparent medical article with viscoelastic properties is particularly advantageous for the treatments of limbs in case of lymphedema or venous insufficiency. The medical article may combine aesthetics, i.e. it may be transparent and thin, protection, i.e. from water and bacteria, and efficacy. The transparent properties of the medical article secure that skin integrity is well observable at any time even in case of longer therapies that go over several weeks.

According to a further aspect of the invention, the adhesive is a skin-friendly adhesive, i.e. the adhesive facilitates easy and gentle removal of the continuous compression film from the skin.

According to an aspect of the invention, the continuous compression film may comprise a polyurethane or similar material. However, alternatively or additionally, the medical article may also comprise other suitable materials with viscoelastic properties known by the person of skill that may be used to form thin compression films. The continuous compression film may comprise and/or may be formed of a variety of materials that meet the limitations of the present disclosure, including, but not limited to, polyurethanes, polyureas, polyethers, polyesters, poly (meth)acrylates, polyolefins, polyvinyl chloride, and combinations thereof.

According to an aspect of the invention, the adhesive may substantially cover the first surface of the continuous compression film. In this aspect, the adhesive may basically form an adhesive layer covering the surface of the continuous compression film. Hence, the medical article is configured in a substantially two-layer configuration.

According to an aspect of the invention, the adhesive may comprises at least one of a pressure-sensitive adhesive and a hypoallergenic adhesive.

According to a further aspect of the invention, the medical article may be particularly configured to protect skin from bacteria.

A pressure-sensitive adhesive is particularly suited for a skin-adhering bandage that is self-adhering in the sense that the medical article just has to be pressed slightly against skin, e.g. of a limb, to achieve a bonding between the continuous compression film and the skin via the adhesive. Suitable adhesives may be particularly selected from the group of acrylates, polyacrylates, polyvinyl ethyl ethers, silicones, or others.

According to an aspect of the invention, at least one of a second surface of the continuous compression film and the adhesive may be surface-treated using at least one of a chemical surface treatment and a physical surface treatment. The surface treatment may be adapted to reduce adhesion of the medical article to itself, that is the adhesion of the second surface of the continuous compression film to the adhesive on the first surface of the continuous compression film, e.g. in case the medical article is wound up in a roll. The chemical surface treatment may comprise at least one of a chemical priming and a chemical coating. The physical surface treatment may comprise at least one of a laser treatment, a plasma treatment, a corona treatment, a flaming treatment and a mechanical roughening treatment. A corona treatment makes use of discharges between electrodes to activate a surface, e.g. to increase the adhesion of plastic and/or polymer surfaces. An alternative method of activating a surface employs a highly accelerated plasma provided from a plasma nozzle.

In addition to, or instead of, a surface treatment, a surface may be roughened by mechanical and/or other methods known to the person of skill. This may be used to increase or decrease the adhesive properties of the respective surface. In a similar vein, surfaces may also be affected by chemical surface treatments. For example, a surface may be coated with an adhesive or non-adhesive material, increasing or decreasing the adhesive properties of a surface. The person of skill will conceive appropriate and suitable methods to affect a surface in either direction, that is to increase or decrease its adhesive capabilities. For example, the polar part of a surface may be affected with such a method, i.e. that part of the surface free energy or the surface tension, which is due to polar interactions. The surface energy or the surface tension of the material may be investigated and/or determined for example by measuring the contact angle of a test liquid on the surface, i.e. the angle between the surface of the liquid and the outline of the contact surface.

According to an aspect of the invention, the medical article may further comprise a liner covering at least one of the continuous compression film and the adhesive. The liner may cover at least a portion of the adhesive. Even though one or several liners are not required in the present disclosure, it may be advantageous for specific applications or configurations of the medical article to include a liner within the medical article. The liner may be for example a thin polyethylene foil or similar that may be wound up with the continuous compression film together in a roll. The liner may be attached to the continuous compression film using an adhesive. Alternatively, the liner may be a non-adhering liner that is positioned and/or placed adjacent to the medical article in the roll. Independent of the presence of a liner, the medical article may be wound up under pre-elongation or without pre-elongation.

Where appropriate, the above-mentioned configurations and developments can be combined in any manner. Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- Fig. 1: schematically illustrates a perspective view of a medical article according to an embodiment of the invention; and
- Fig. 2: schematically illustrates a perspective view of a skin-adhering bandage according to an embodiment of the invention comprising the medical article of Fig. 1.

The accompanying drawings are included to provide a further understanding of the present invention and are incorporated in and constitute a part of this specification. The drawings illustrate the embodiments of the present invention and together with the description serve to explain the principles of the invention. Other embodiments of the present invention and many of the intended advantages of the present invention will be readily appreciated as they become better understood by reference to the following detailed description. The elements of the drawings are not necessarily to scale relative to each other.

In the figures, like reference numerals denote like or functionally like components, unless indicated otherwise.

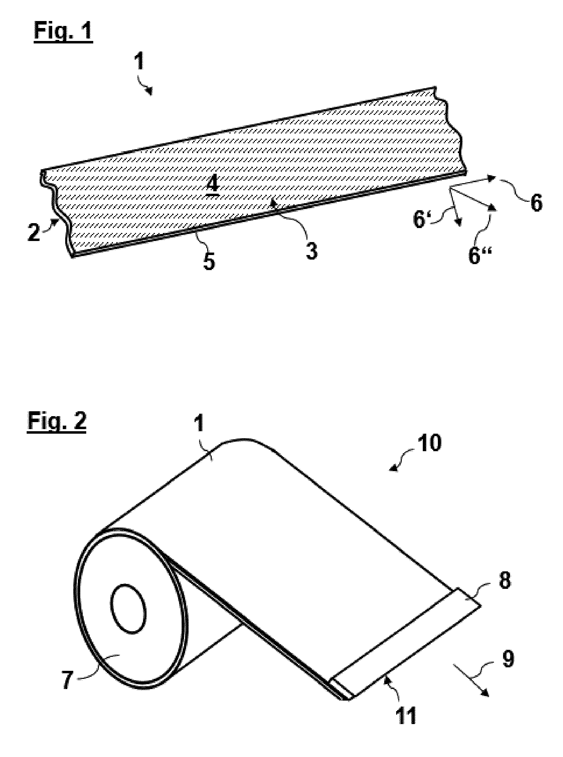

Figure 1 schematically illustrates a perspective view of a medical article according to an embodiment of the invention.

In Figure 1, reference sign 1 denotes a medical article according to the invention. The medical article 1 may form at least a portion of a skin-adhering bandage 10 for compression therapy (cf. Fig. 2). However, in a similar vein the medical article 1 may also form a portion and/or be part of compression wraps, compression dressings, compression devices or other similar devise and/or articles used in compression therapy. Such compression devices may be used in compression therapy for applying compression to a body part (e.g. a limb, torso, head, neck and so on) of a subject for the use in the treatment and/or management of chronic wounds, edema and other venous and lymphatic disorders of a body part, more particularly venous leg ulcers and lymphedema of a limb. In the following, the utilization of the medial article 1 within a compression bandage 10 will be detailed. The person of skill will however readily acknowledge that the present teachings may be appropriately adapted to other applications in a straightforward way.

The medical article 1 in Fig. 1 has the general shape of a band or strap or ribbon. Consequently a length of the medical article 1 may be defined as the dimension of the medical article 1 in the length direction of the band or ribbon. A width of the medical article 1 may be similarly defined as the dimension of the medical article 1 perpendicular to the length direction within the plane of the medical article 1. Finally, a thickness of the medical article 1 may be defined as the dimension of the medical article 1 in a direction oriented orthogonally with respect to the plane of the medical article 1. In exemplary embodiments, the medical article thus may have a length of a few decimeters up to several meters, a width between 5 cm and 20 cm, and a thickness of less than 40 µm. In one exemplary embodiment, the medical article 1 has a length of one meter, a width of 10 cm, and a thickness of 30 µm.

The medical article 1 in Fig. 1 comprises a continuous compression film 2 for application around the limb (not shown) to provide therapeutic compression of the limb. The term "continuous" generally refers to the continuous compression film 2 being substantially continuous such that the layer does not includes voids, openings, cutaway regions, or regions of discontinuity. To provide compression of the limb, the continuous compression film 2 is configured with viscoelastic properties in a stretch direction 6 in the plane of the continuous compression film 2. The stretch direction 6 is the length direction of the medical article 1. The stretch direction 6 substantially corresponds to an application direction 9 of the medical article 1 (cf. Fig. 2). However, other continuous compression films 2 may also have viscoelastic properties in a second stretch direction 6', which is oriented perpendicular to the first stretch direction 6 along a width of the continuous compression film 2. More generally, the continuous compression film 2 may have viscoelastic properties in one or several directions 6, 6' being oriented within a plane of the continuous compression film 2.

To achieve the mentioned viscoelastic properties, the continuous compression film 2 comprises a polyurethane. It is contemplated that the continuous compression film 2 may also comprise and/or may be formed of polyurethanes, polyureas, polyethers, polyesters, poly (meth)acrylates, polyolefins, polyvinyl chloride, or combinations thereof.

The medical article 1 further comprises an adhesive 3 substantially covering a first surface 4 of the continuous compression film 2. The medical article 1 is thus configured with two general layers, namely the continuous compression film 2 and the adhesive 3. However, the present disclosure also includes embodiments, where the adhesive 3 does not form a layer or film, but may, for example, be only applied to portions of the first surface 4 of the continuous compression film 2.

The adhesive 3 is configured such as to skin-attach the continuous compression film 2, i.e. attach the film 2 to skin. For this purpose it comprises a pressure-sensitive adhesive. The medical article 1 can thus be pressed slightly against the skin of a limb to achieve a bonding between the continuous compression film 2 and the skin via the adhesive. The adhesive 3, as well as the continuous compression film 2, are transparent, facilitating visual inspection of the skin beneath the applied medical article 1.

Due to the viscoelasticity of the continuous compression film 2, the medical article 1 particularly features a strain-rate dependent tensile modulus in the stretch direction 6. In particular, the tensile modulus increases with increasing strain rate in the stretch direction 6. This property of the continuous compression film 2 allows the medical article 1 to exhibit a stiffer behavior in response to higher strain rates and a softer behavior in response to lower strain rates. Examples of higher strain rates may occur, for example, when the patient is moving his limb. Lower strain rates may occur, for example, as a result of allowing the subject to lie, to stand, or to sit, to have decreasing edema and so on. Due to these properties, the medical article 1 is able to adapt to body contours providing an excellent close fit with low pressure in a supine position. Furthermore, the medical article 1 is able to adapt to shrinking limb contours, e.g. in case of edema reduction. At the same time, the medical article 1 is stiff against rapid movements and/or high strain rates. Hence, a high static stiffness index is achieved, which consequently implies a good venous muscle pump efficacy.

Figure 2 schematically illustrates a perspective view of a skin-adhering bandage 10 according to an embodiment of the invention comprising the medical article 1 of Fig. 1. The medical article 1 is shown in Fig. 2 in a rolled form or configuration, i.e. it is wound up in a roll 7. During application, the medical article 1 can be unrolled directly onto the skin of a body part of a subject into an application direction 9. To this end, the medical article 1 may be grabbed at an application end 11 of the medical article 1 and pulled into the application direction 9. To ease the application of the medical article 1, a liner tab 8 is attached to the application end 11 of the medical article 1 in the application direction 9. The liner tab 8 facilitates grabbing such that the edges of the medical article keep uniformly stretched during application. The liner tab 8 comprises a rigid plastic material. Alternatively, the liner tab may also be configured as an elastic and/or viscoelastic film or layer, e.g. a foil or the like. In one embodiment, the liner tab 8 covers 2 cm of the adhesive 3 in the application direction 9 over substantially the full width of the medical article 1.

The medical article 1 is rolled up under a pre-elongation in the application direction 9. The pre-elongation in the application direction 9 amounts to 170% of the original length of the medical article 1 in the application direction 9. The medical article 1 is thus elongated to the desired stretch ratio and rolled up while stretched, such that it maintains its stretch ratio.

The strain-rate-dependent tensile modulus of the medical article 1 of the present disclosure enables novel methods of applying the skin-adhering bandage 10. The specific characteristic of the skin-adhering bandage 10 of the present disclosure is that its unconstrained strain recovery is slow compared to similar articles comprising only elastomers with less strain-rate dependence. As mentioned above, the skin-adhering bandage 10 of the present disclosure can be elongated to a desired stretch ratio and rolled up while stretched such that it maintains its stretch ratio. To reduce self-adherence and avoid difficulty in unwinding, the medical article 1 further includes an agent to reduce self-adhesion of the medical article 1. Specifically, the medical article 1 comprises a chemical coating on a second surface 5 of the continuous compression film 2 that reduces self-adherence of the medical article 1 to itself, i.e. the adhesion of the adhesive 3 to the continuous compression film 2. Alternatively, an additional non-adherent layer or liner (not shown) may be wound with the medical article 1. The medical article 1 can be unrolled directly onto the body part of a subject with negligible application of tension as the skin-adhering bandage 10 is unrolled. Within seconds, the medical article 1 will begin to recover its original length, contract and thereby apply the amount of pressure to the patient that is determined by the stretch ratio applied to the medical article 1 before it was wound up. Because the medical article 1 and the skin-adhering bandage 10 of the present disclosure can be applied with negligible tension and still maintain the correct stretch ratio, it can properly be applied by a less-skilled caregiver. Further, the medical article 1 and the skin-adhering bandage 10 of the present disclosure can be manufactured in very thin configurations, thus saving cost and weight. Additional support layers or similar as in conventional compression bandages are not required. As a result, the medical article 1 and the skin-adhering bandage 10 according to the invention are inexpensive, easy to use, and combine efficacy with a high level of comfort.

Although specific embodiments are illustrated and described herein, it will be appreciated by those of ordinary skill in the art that a variety of alternate and/or equivalent implementations may be substituted for the specific embodiments shown and described without departing from the scope of the present invention. Generally, this application is intended to cover any adaptations or variations of the specific embodiments discussed herein.

The embodiments were chosen and described in order to best explain the principles of the invention and its practical applications, to thereby enable others skilled in the art to best utilize the invention and various embodiments with various modifications as are suited to the particular use contemplated. Many other examples will be apparent to one skilled in the art upon reviewing the above specification.

## Claims

1. Medical article (1) for use in compression therapy of a patient's limb, comprising a continuous compression film (2) for application around the limb to provide compression of the limb and an adhesive (3) covering at least a portion of a first surface (4) of the continuous compression film (2), the adhesive (3) being configured such as to skin-attach the continuous compression film (2), wherein the continuous compression film (2) has viscoelastic properties in at least one stretch direction (6, 6') in the plane of the continuous compression film (2).

2. Medical article (1) according to claim 1, wherein the medical article (1) has, in the stretch direction (6, 6'), a tensile modulus, which increases with increasing strain rate.

3. Medical article according to any one of the preceding claims, wherein the continuous compression film (2) has, for a sinusoidal deformation of the continuous compression film (2) into the stretch direction (6, 6', 6") in a dynamic mechanical analysis, a phase lag of δ between strain and stress, a storage modulus E' and a loss modulus E", wherein tan δ = E"/E' > 0.3 at maximum as a function of temperature.

4. Medical article according to claim 3, wherein the maximum of tan δ defines the glass transition temperature of the continuous compression film (2), the glass transition temperature having values between 15 °C and 55 °C.

5. Medical article according 4, wherein, at the glass transition temperature, the storage modulus E' has values between 5 MPa and 500 MPa.

6. Medical article according to any one of the preceding claims, wherein the medical article (1) is wound up in a roll (7) into an application direction (9) of the medical article (1).

7. Medical article according claim 6, wherein the medical article (1) is wound up under a pre-elongation in the application direction (9).

8. Medical article according claim 7, wherein the pre-elongation in the applica-tion direction (9) amounts to 120% to 250%, preferably 150% to 190%, of original length of the medical article (1) in the application direction (9).

9. Medical article according to any one of the claims 6 to 8, further comprising a liner tab (8) attached to at least one application end (11) of the medical article (1) in the application direction (9).

10. Medical article according to any one of the preceding claims, wherein the medical article (1) has a thickness of less than 40 µm in a non-stretched state.

11. Medical article according to any one of the preceding claims, wherein at least one of the continuous compression film (2) and the adhesive (3) comprises a visually transparent portion.

12. Medical article according to any one of the preceding claims, wherein:
- the continuous compression film (2) comprises a polyurethane, and/or
- the adhesive (3) substantially covers the first surface (4) of the continuous compression film (2) and/or
- the adhesive (3) comprises at least one of a pressure-sensitive adhesive and a hypoallergenic adhesive.

13. Medical article according to any one of the preceding claims, wherein at least one of a second surface (5) of the continuous compression film (2) and the adhesive (3) is surface-treated using at least one of a chemical surface treatment and a physical surface treatment, wherein the chemical surface treatment comprises at least one of a chemical priming and a chemical coating, and wherein the physical surface treatment comprises at least one of a laser treatment, a plasma treatment, a corona treatment, a flaming treatment and a mechanical roughening treatment.

14. Medical article according to any one of the preceding claims, further comprising a liner covering at least one of the continuous compression film (2) and the adhesive (3).

15. Skin-adhering bandage (10) for compression therapy, comprising a medical article (1) according to any one of the preceding claims.
